# EUROPÄISCHE PATENTSCHRIFT

(11) **EP 0 966 427 B1**
(45) Veröffentlichungstag und Bekanntmachung des Hinweises auf die Patenterteilung: **02.05.2001**
(21) Anmeldenummer: 98912276.7
(22) Anmeldetag: 18.02.1998
(51) Int. Cl.: C07C 231/02, C07D 487/22, A61K 49/00, A61K 47/48, A61K 47/22, A61K 47/30, C07F 15/00, C07F 17/02, C07F 5/06

(54) **HERSTELLUNG VON SÄUREAMIDEN UND METALLIERUNG VON VERBINDUNGEN**
PREPARATION OF ACID AMIDES AND METALLISATION OF COMPOUNDS
PREPARATION D'AMIDES D'ACIDES ET METALLISATION DE COMPOSES

(30) Priorität: 19.02.1997 DE 19706490
(43) Veröffentlichungstag der Anmeldung: 29.12.1999
(73) Patentinhaber: Deutsches Krebsforschungszentrum Stiftung des öffentlichen Rechts, 69120 Heidelberg (DE)
(72) Erfinder: SINN, Hannsjörg, D-69118 Wiesloch (DE); MAIER-BORST, Wolfgang, D-69221 Dossenheim (DE); SCHRENK, Hans-Hermann, D-67728 Zeiskam (DE); STEHLE, Gerd, D-69123 Wieblingen (DE)
(74) Vertreter: Schüssler, Andrea, Dr.
(86) Internationale Anmeldenummer: DE9800496
(87) Internationale Veröffentlichungsnummer: WO9837057

(56) Entgegenhaltungen:
- WO-A-90/03804
- WO-A-91/18630
- WO-A-96/32134
- GB-A- 775 005
- GB-A- 1 054 044
- US-A- 4 826 673

## Beschreibung

Die Erfindung betrifft Verfahren zur Herstellung von Säureamiden und die Metallierung von zur Bindung mit einem Metall oder Metallion fähigen Verbindungen, die dadurch erhaltenen Produkte und ihre Verwendung zur Therapie und/oder Diagnose von Tumoren oder entzündlichen Erkrankungen.

Es ist bekannt, zur Bindung mit einem Metallion fähige Verbindungen, z.B. Diethylentriaminpentaessigsäure (DTPA), Porphyrine, Phthalocyanine und Naphthalocyanine zur Therapie und/oder Diagnose von Tumoren einzusetzen. Um diese Verbindungen im Tumor anzureichern und die Eliminationsgeschwindigkeit aus dem Körper des Patienten zu vermindern, sind an diese Verbindungen höhermolekulare Substanzen, z.B. Polyethylenglykole, gebunden. Zur Bindung der höhermolekularen Substanzen wird die Ausbildung von Säureamidbindungen ausgenutzt. Allerdings gelingt die direkte Umsetzung einer Säure mit einem Amin nicht. Daher wird die Säure, z.B. Gd³⁺-enthaltendes DTPA (Gd-DTPA) oder Tetrasulfophthalocyanin (TSPC), zuerst unter Verwendung eines Halogenierungsmitteis in ein Säurehalogenid umgewandelt. Überschüssiges Halogenierungsmittel wird dann abgetrennt, was jedoch oft nicht vollständig oder nur in sehr aufwendiger Weise möglich ist. Das Säurehalogenid wird dann gereinigt. Anschließend wird es mit einem Amin, z.B. Amino -Ω- methoxy-polyethylenglykol (AMPEG) zum Säureamid umgesetzt. Das Säureamid muß dann gereinigt werden. Somit ist dieses Verfahren nur mit großem Aufwand durchführbar.

An vorstehende Verbindungen (DTPA, Porphyrine etc.) können Metallionen gebunden sein. Hierzu ist allerdings oftmals eine lange Umsetzungszeit zwischen den Verbindungen und dem Metallion notwendig. Auch wird das Produkt oftmals nur mit geringer Ausbeute erhalten.

Der Erfindung liegt somit die Aufgabe zugrunde, ein Verfahren bereitzustellen, mit dem Säureamide, die ggf. ein Metallion aufweisen, einfach, schonend und schnell hergestellt werden können.

Erfindungsgemäß wird dies durch die Gegenstände in den Patentansprüchen erreicht.

Gegenstand der vorliegenden Erfindung ist somit ein Verfahren zur Herstellung von Säureamiden, bei dem eine Säure mit einem Amin in einer Harnstoffschmelze umgesetzt wird.

Der Ausdruck "Säure" umfaßt Säuren jeglicher Art, insbesondere Carbon-, Sulfon- und Phosphonsäuren. Die Säure kann auch mehrere Säuregruppen aufweisen, z.B. 2 bis 4. Bei mehreren können diese gleich oder verschieden voneinander sein. Bevorzugte Säuren sind Diethylentriaminpentaessigsäure (DTPA), Diethylentriaminpentamethylenphosphonsäure (DTPMPA), ein Säuregruppen-aufweisendes Porphyrin, wie Tetrasulfophenylporphyrin (TSPP) und Tetracarboxyphenylporphin(TCPP), ein Säuregruppen-aufweisendesPhthalocyanin, wie Tetrasulfophthalocyanin (TSPC), ein Säuregruppen-aufweisendes Naphthalocyanin, ein Säuregruppen-aufweisendes Chlorin, ein Säuregruppen-aufweisendes Bakteriochlorin, ein Säuregruppen-aufweisendes Chlorophyll, ein Säuregruppen-aufweisendes Bakteriochlorophyll oder ein Derivat von diesen.

Der Begriff "Porphyrin" umfaßt Verbindungen jeglicher Art, die ein makromolekulares 4- oder mehrkerniges Pyrrolgerüst aufweisen. Vertreter dieser Verbindungen sind Porphyrine, die eine oder mehrere, vorzugsweise 4, funktionelle Säuregruppen aufweisen. Weitere Vertreter sind die expandierten Porphyrine, wie Saphryin, Rubyrin, Pentaphyrin, Superphthalocyanine oder Texaphyrine.

Der Ausdruck "Amin" umfaßt Verbindungen jeglicher Art, die eine aliphatische Aminogruppe aufweisen. Die Amine können primäre oder sekundäre Amine sein. Beispiele von Aminen sind Tris, Glucamin, Glucosamin und Aminopolyether.

Letztere sind Polyether, z.B. ein Polyethylenglykol, mit einer Aminogruppe, insbesondere einer terminalen Aminogruppe. Vorzugsweise weist der Aminopolyether ein Molekulargewicht von 2000 bis 5000 Dalton auf. Ein besonders bevorzugter Aminopolyether ist Amino-Ω-methoxy-polyethylenglykol.

Die Umsetzung der Säure mit dem Amin erfolgt in einer Harnstoffschmelze. Die Harnstoffschmelze fungiert dabei als Lösungsmittel. Die Menge an Harnstoff kann so gewählt werden, daß in der Harnstoffschmelze die Edukte und ggf. auch die Produkte gelöst sind. Bei der Umsetzung wird das Amin in einem Überschuß, z.B. von 50%, insbesondere 20%, bezogen auf die Menge an Säuregruppen, an die das Amin binden soll, verwendet. Die Reaktionstemperatur beträgt 120 bis 170 °C, besonders bevorzugt ca. 150 bis 160 °C. Die Reaktion ist beendet, wenn eine im wesentlichen quantitative Umsetzung erfolgt ist. Dies kann von einem Fachmann leicht mit üblichen spektroskopischen und/oder chromatographischen Verfahren festgestellt werden. Üblicherweise beträgt die Reaktionszeit 1 Minute bis 12 Stunden, insbesondere 10 bis 20 Minuten, ganz besonders etwa 15 Minuten. Für das vorstehende Verfahren eignen sich jegliche in der Chemie verwendete Gefäße, die bei diesen Reaktionsbedingungen eingesetzt werden können. Besonders günstig ist es, die Reaktion in einem verschlossenen Glasfläschchen durchzuführen. Das Erwärmen kann z.B. in einem Wasserdampf-autoklav erfolgen.

Die Isolierung des Säureamids kann dadurch erfolgen, daß der Reaktionsansatz ggf. nach Abkühlen in einem Lösungsmittel aufgenommen wird. Als günstige Lösungsmittel haben sich wäßrige Lösungsmittel, wie Wasser und saure oder alkalische Puffer, wie 0,17 M NaHCO₃, erwiesen, in denen sich der Harnstoff gut löst. Das Lösungsmittel kann so gewählt werden, daß die Produkte darin gelöst werden. Danach kann das Säureamid in üblicher Weise, insbesondere durch Ultrafiltration, gereinigt werden. Das Säureamid kann getrocknet, z.B. gefriergetrocknet, oder in einem Lösungsmittel, wie Wasser, Dioxan, Dimethylformaid (DMF), Dimethylacetamid (DMAA), Dimethylsulfoxid (DMSO) und Dimethylpropylharnstoff (DMPH), gelöst werden.

Gegenstand der vorliegenden Erfindung ist auch ein Verfahren zur Metallierung von zur Bindung mit einem Metallion fähigen Verbindung, welches das Umsetzen der Verbindung mit einem Metallion in einer Harnstoffschmelze umfaßt.

Die "zur Bindung mit einem Metallion fähige Verbindung" kann erfindungsgemäß Diethylentriaminpentaessigsäure Diethylentriaminpentamethylenphosphonsäure, ein Porphyrin, ein Phthalocyanin, ein Naphthalocyanin, ein Chlorin, ein Bakteriochlorin, ein Chlorophyll, ein Bakteriochlorophyll oder ein Derivat von diesen sein.

Vorzugsweise ist die zur Bindung eines Metallions fähige Verbindung eine Säure (vgl. vorstehend). Die Bindung kann eine Komplexbindung oder eine lonenbindung sein. Die Bindung kann z.B. über 4 im wesentlichen in einer Ebene angeordnete Stickstoffatome, die zu einem Ringsystem verbunden sind, oder über mehrere, z.B. 3, vorstehende Säuregruppen erfolgen.

An eine solche Verbindung kann ein Metallion gebunden sein. Beispiele von Metallionen sind Ionen von Al, Mg, Zn, Cu, Co, Fe, Ni, Pt, Pd, Gd, Ga und In. Beispiele von Metallionen sind insbesondere Mg²⁺, Cu²⁺, Zn²⁺, Ga³⁺, Co³⁺, Al³⁺, Zn⁴⁺, Pt⁴⁺, Pd⁴⁺ und Gd³⁺. Das Metallion kann radioaktiv sein. Es ist günstig, die Radien der Metallionen so zu wählen, daß sie optimal in die Struktur der Verbindung passen. Werden Porphyrine, Phthalocyanine, Naphthalocyanine, Chlorine, Bakteriochlorine, Chlorophyll oder Bakteriochlorophyll verwendet, dann ist der Radius günstigerweise kleiner als 0,76 Å. Werden Metallionen zur Metallierung eingesetzt, dann werden diese günstigerweise als Salze, z.B. Hydroxide, zum Reaktionsansatz zugegeben.

Wie vorstehend beschrieben kann die Säure eine zur Bindung eines Metallions fähige Verbindung sein. An diese kann ein Metallion gebunden sein. Die Bindung des Metallions kann während, vor oder nach der Umsetzung der Säure mit dem Amin zum Säureamid erfolgen, wobei die Bindung in einer Harnstoffschmeize erfolgen kann. Dies kann dabei unter im wesentlichen den gleichen Bedingungen erfolgen, wie die Säureamidbildung in der Harnstoffschmelze.

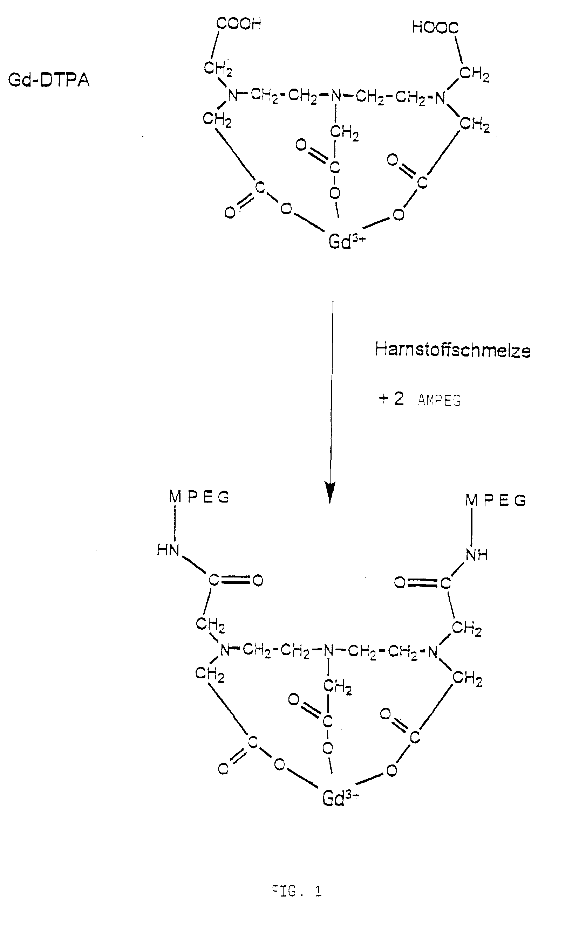

Besonders bevorzugte Verfahren sind in den Figuren 1 bis 6 dargestellt.

Der Gegenstand der vorliegenden Erfindung zeichnet sich durch eine Reihe von Vorteilen aus. Das Verfahren führt in einem einzigen Reaktionschritt ohne zwischengeschaltete Reinigungsschritte zu den Produkten.Dabei ist die Umsetzung nach kurzer Zeit, insbesondere nach ca. 15 Minuten, quantitativ erfolgt. Ferner ist kein Verlust eines mit der Säure komplexierten Metalls oder Metallions zu beobachten. Auf diese Weise können in direkter Reaktion wasserlösliche makromolekulare Verbindungen von DTPA-, Porphyrin-, Phtalocyanin- und Naphthalocyanin-Komplexen hergestellt werden, die sowohl für die Tumordiagnostik als auch für die Tumortherapie von Bedeutung sind. Diese Verbindungen waren bisher gar nicht oder nur unter großem Aufwand zugänglich.

Kurze Beschreibung der Zeichnungen:
- Figur 1:: zeigt die Herstellung eines Säureamids aus Gd-DTPA und AMPEG,
- Figur 2:: zeigt die Herstellung eines Säureamids aus Gd-DTPMPA und AMPEG,
- Figur 3:: zeigt die Herstellung eines Säureamids aus TSPP und AMPEG,
- Figur 4:: zeigt die Herstellung eines Säureamids aus TSPC und AMPEG,
- Figur 5:: zeigt die Metallierung von TCPP mit Mg²⁺ und
- Figur 6:: zeigt die gleichzeitige Metallierung und Säureamidbildung.

Die folgenden Beispiele erläutern die Erfindung:

### Beispiel 1: Herstellung eines Säureamids aus Gd-DTPA und AMPEG

Die Herstellung und das erhaltene Säureamid sind in Fig. 1 dargestellt.

50 mg GD-DTPA (0,09 mM) wurden zusammen mit etwa 3,5 g Harnstoff und 1,2 g AMPEG (0,24 mM/MG ~ 5000) in einem 15 ml Glasfläschchen mit Bördelrandverschlußvorgelegt, mit einem Silicongummistopfen und einer Aluminiumkappe fest verschlossen und in einem Wasserdampf-Autoklav 15 min auf 155 °C erhitzt.

Nach dem Abkühlen auf etwa 105°C wurde der Autoklav belüftet, das Reaktionsgefäß entnommen und die zumeist erstarrte Harnstoffschmelze in 10 ml dest. Wasser gelöst. Die klare Lösung wurde zur Reinigung und Abtrennung unerwünschter Begleitstoffe in eine Ultrafiltrationszelle überführt und über ein Ultrafilter (Amicon, YM5)4-5 mal gewaschen (Abreicherungsfaktor >20x10⁵). Es wurde ein Säureamid aus Gd-DTPA und AMPEG erhalten. Dieses kann lyophilisiert werden, in Wasser mit beliebigen pH oder in organischen Lösungsmittel (DMF, DMAA, DMSO, DMPH) gelöst werden.

### Beispiel 2: Herstellung eines Säureamids aus GD-DTPMPA und AMPEG

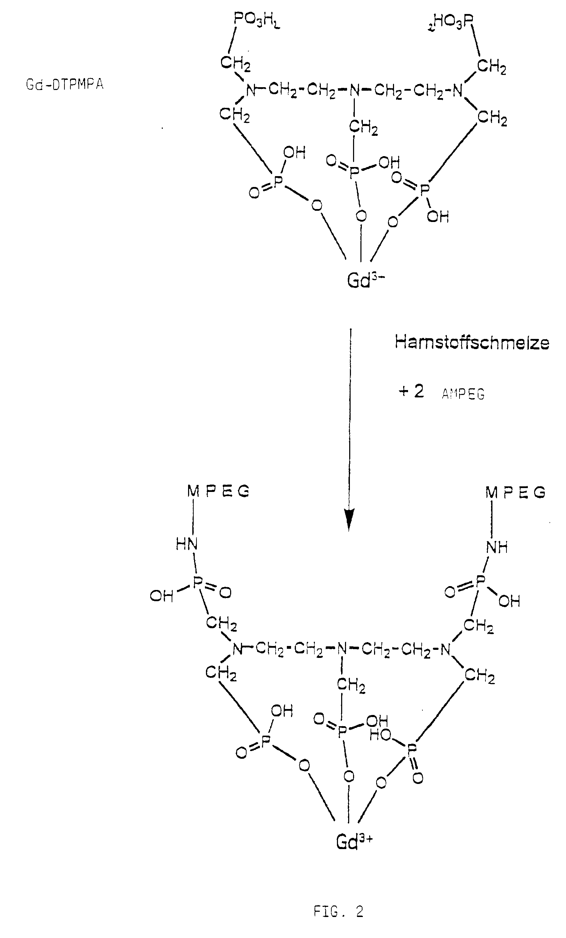

Die Herstellung und das erhaltene Säureamid sind in Fig. 2 dargestellt.

Es wurde das gleiche Verfahren wie in Beispiel 1 angewandt, mit der Ausnahme, daß anstatt DTPA DTPMPA verwendet wurde.

### Beispiel 3: Herstellung eines Säureamids aus TSPP und AMPEG

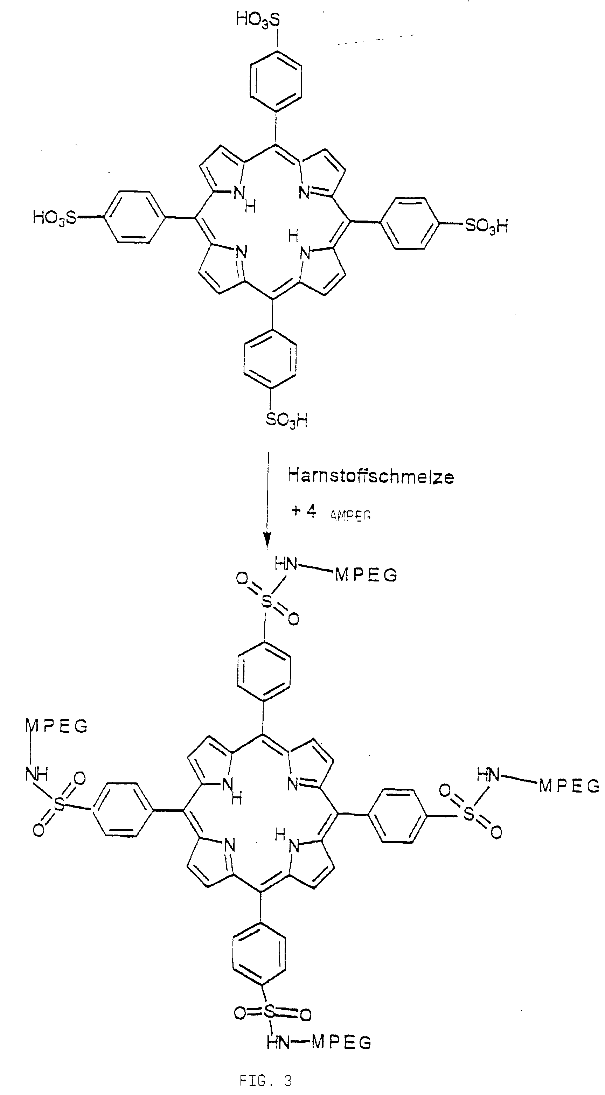

Die Herstellung und das erhaltene Säureamid sind in Fig. 3 dargestellt.

Etwa 40 mg TSPP (Tetra-(4-sulfophenyl)Porphin, MG ~ 934, Porphyrin Products) (0,043 mM) wurden zusammen mit etwa 3,5 g Harnstoff und 1,2 g AMPEG (0,24 mM) in einem 15 ml Glasfläschchen mit Bördelrandverschluß vorgelegt, mit einem Silicongummistopfen und einer Aluminiumkappe fest verschlossen und in einem Wasserdampf-Autoklav 15 min auf 155°C erhitzt.

Nach dem Abkühlen auf etwa 105°C wurde der Autoklav belüftet, das Reaktionsgefäß entnommen und die zumeist erstarrte Harnstoffschmelze in 10 ml dest. Wasser gelöst. Die klare, tief rote Lösung wurde zur Reinigung und Abtrennung unerwünschter Begleitstoffe in eine Ultrafiltrationszelle überführt und über ein Ultrafilter (Amicon, YM 10)4-5 mal gewaschen (Abreicherungsfaktor > 1x10⁵). Es wurde ein Säureamid aus TSPP und AMPEG erhalten. Dieses kann lyophilisiert werden, in Wasser mit beliebigem pH oder in organischen Lösungsmitteln (Dioxan, DMF, DMAA, DMSO, DMPH) gelöst werden.

### Beispiel 4: Herstellung eines Säureamids aus TSPC und AMPEG

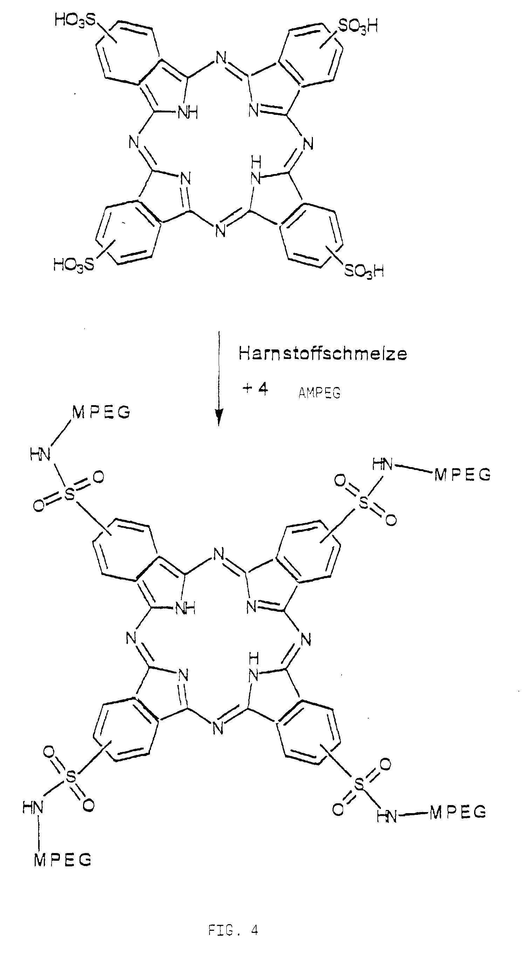

Die Herstellung und das Säureamid sind in Fig. 4 dargestellt.

Etwa 40 mg TSPC (Tetra-Sulfophthalocyanin, MG ~ 836, Porphyrin Products) (0,048 mM) wurden zusammen mit etwa 3,5 g Harnstoff und 1,2 g AMPEG (0,24 mM) in einem 15 ml Glasfläschchen mit Bördelrandverschluß vorgelegt, mit einem Silicongummistopfen und einer Aluminiumkappe fest verschlossen und in einem Wasserdampf-Autoklav 15 min auf 155°C erhitzt.

Nach dem Abkühlen auf etwa 105°C wurde der Autoklav belüftet, das Reaktionsgefäß entnommen und die zumeist erstarrte Harnstoffschmelze in 10 ml dest. Wasser gelöst. Die klare, tief blaue Lösung wurde zur Reinigung und Abtrennung unerwünschter Begleitstoffe in eine Ultrafiltrationszelle überführt und über ein Ultrafilter (Amicon, YM 10) 4-5 mal gewaschen (Abreicherungsfaktor > 2x10⁵). Es wurde ein Säureamid aus TSPC und AMPEG erhalten. Dieses kann lyophilisiert werden, in Wasser mit beliebigem pH oder in organischen Lösungsmitteln (Dioxan, DMF, DMAA, DMSO, DMPH) gelöst werden.

### Beispiel 5: Metallierung von TCPP mit Mg²⁺

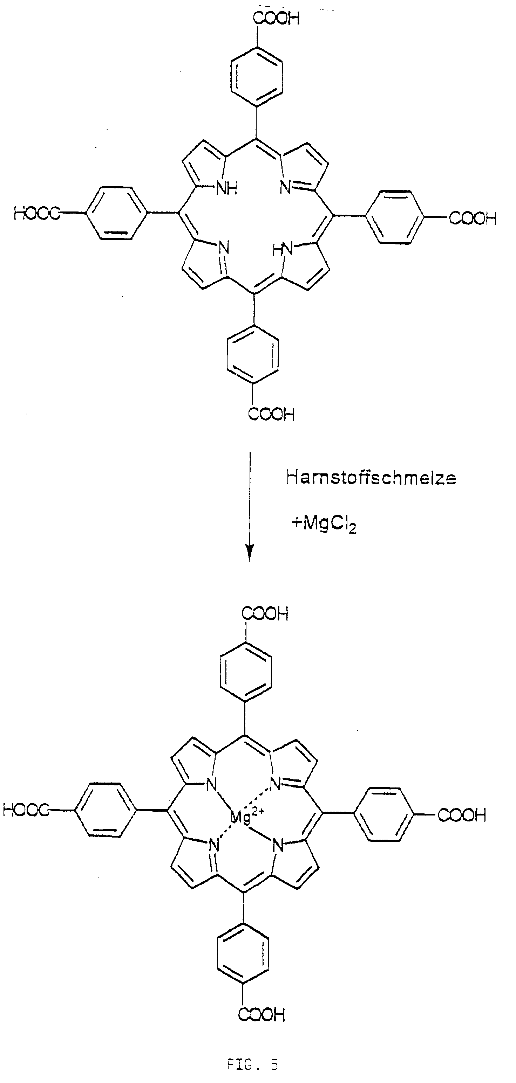

Die Herstellung und der erhaltene Komplex sind in Fig. 5 dargestellt.

Etwa 40 mg TCPP (Tetra-(4-Carboxyphenyl)Porphin, MG = 790) (0,05 mM) wurden zusammen mit etwa 3,5 g Harnstoff und 70 mg Mg(OH)₂ (1,2 mM) in einem 15 ml Glasfläschchen mit Bördelrandverschluß vorgelegt, mit einem Silicongummistopfen und einer Aluminiumkappe fest verschlossen und in einem Wasserdampf-Autoklav 15 min auf 155°C erhitzt.

Nach dem Abkühlen auf etwa 105°C wurde der Autoklav belüftet, das Reaktionsgefäß entnommen und die zumeist erstarrte Harnstoffschmelze in 10 ml 0,17 M NaHCO₃ Lösung gelöst. Die klare, dichroitische Lösung wurde zur Reinigung und Abtrennung unerwünschter Begleitstoffe in eine Ultrafiltrationszelle überführt und über ein Ultrafilter (Amicon, YC 05)4-5 mal gewaschen (Abreicherungsfaktor >2x10⁵). Es wurde ein Komplex aus TCPP und Mg²⁺ erhalten. Dieser kann durch Gefriertrocknung isoliert werden und anschließend mit Aminen wie z.B.: Tris, Glucamin, Glucosamin, MPEG etc. in einer Harnstoffschmelze zum Säureamid umgesetzt werden.

### Beispiel 6: Gleichzeitige Metallierung und Säureamidbildung

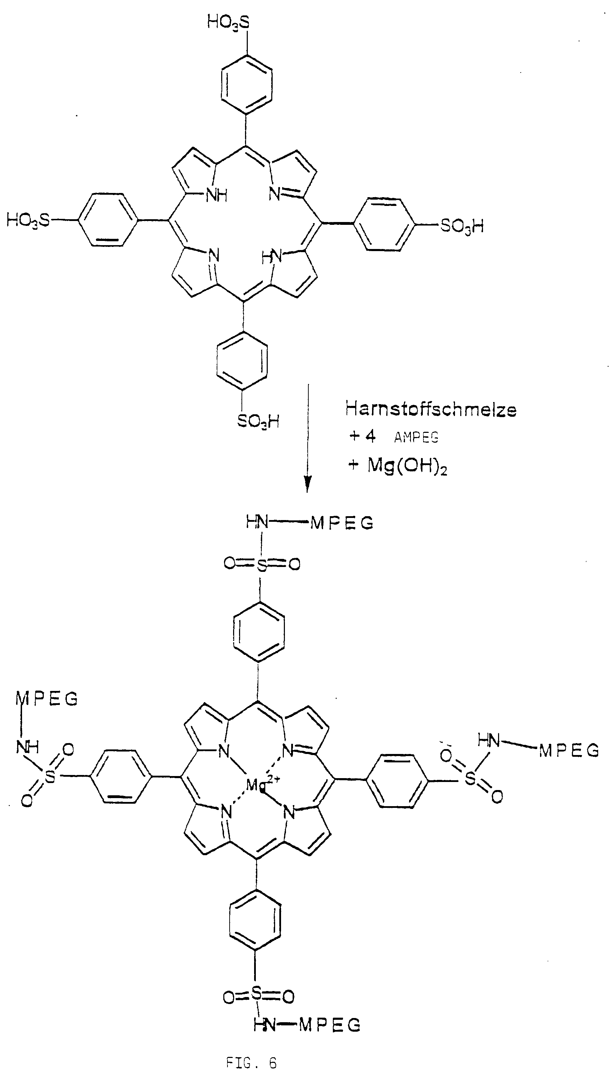

Die Reaktion ist in Fig. 6 dargestellt.

Etwa 40 mg TSPP (Tetra-(4-sulfophenyl)Porphin, MG ~ 934) (0,043 mM) wurden zusammen mit etwa 3,5 g Harnstoff, 70 mg Mg(OH)₂ (1,2 mM) und 1,2g AMPEG (0,24 mM) in einem 15 ml Glasfläschchen mit Bördelrandverschluß vorgelegt, mit einem Silicongummistopfen und einer Aluminiumkappe fest verschlossen und in einem Wasserdampf-Autoklav 15 min auf 155°C erhitzt.

Nach dem Abkühlen auf etwa 105°C wurde der Autoklav belüftet, das Reaktionsgefäß entnommen und die zumeist erstarrte Harnstoffschmelze in 10 ml dest. Wasser gelöst. Die klare, dichroitische Lösung wurde zur Reinigung und Abtrennung unerwünschter Begleitstoffe in eine Ultrafiltrationszelle überführt und über ein Ultrafilter (Amicon, YM 10)4-5 mal gewaschen (Abreicherungsfaktor > 2x10⁵). Das Produkt kann lyophilisiert werden, in Wasser mit beliebigem pH oder in organischen Lösungsmittel (DMF, DMAA, DMSO, DMPH(U)) gelöst werden.

## Patentansprüche

1. Verfahren zur Herstellung von Säureamiden, umfassend das Umsetzen einer Säure mit einem aliphatischen Amin in einer Harnstoffschmelze.

2. Verfahren nach Anspruch 1, dadurch gekennzeichnet, daß die Säure eine Carbon-, Sulfon- oder Phosphonsäure ist.

3. Verfahren nach Anspruch 1 oder 2, dadurch gekennzeichnet, daß die Säure Diethylentriaminpentaessigsäure, Diethylentriaminpentamethylenphosphonsäure, ein Säuregruppen-aufweisendes Porphyrin, ein Säuregruppen-aufweisendes Phthalocyanin, ein Säuregruppen-aufweisendes Naphthalocyanin, ein Säuregruppen-aufweisendes Chlorin, ein Säuregruppen-aufweisendes Bakteriochlorin, ein Säuregruppen-aufweisendes Chlorophyll, ein Säuregruppen-aufweisendes Bakteriochlorophyll oder ein Derivat von diesen ist.

4. Verfahren nach einem der Ansprüche 1-3, dadurch gekennzeichnet, daß das Amin ein primäres oder sekundäres aliphatisches Amin ist.

5. Verfahren nach Anspruch 4, dadurch gekennzeichnet, daß das Amin Tris, Glucamin, Glucosamin oder ein Aminopolyether ist.

6. Verfahren nach einem der Ansprüche 1-5, dadurch gekennzeichnet, daß die Reaktionstemperatur 120 bis 170°C beträgt.

7. Verfahren nach einem der Ansprüche 1-6, dadurch gekennzeichnet, daß die Umsetzung während 1 Minute bis 12 Stunden erfolgt.

8. Verfahren nach einem der Ansprüche 1-7, weiterhin umfassend die Umsetzung der Säure mit einem Metallion in einer Harnstoffschmelze.

9. Verfahren zur Metallierung von einer zur Bindung mit einem Metallion fähigen Verbindung, umfassend die Umsetzung der Verbindung mit einem Metallion in einer Harnstoffschmelze, dadurch gekennzeichnet, daß die Verbindung Diethylentriaminpentaessigsäure, Diethylentriaminpentamethylenphosphonsäure, ein Porphyrin, ein Phthalocyanin, ein Naphthalocyanin, ein Chlorin, ein Bakteriochlorin, ein Chlorophyll, ein Bakteriochlorophyll oder ein Derivat von diesen ist.

10. Verfahren nach Anspruch 9, dadurch gekennzeichnet, daß die zur Bildung eines Metallions fähige Verbindung eine Säure ist.

11. Verfahren nach einem der Ansprüche 8-10, dadurch gekennzeichnet, daß das Metallion ein Ion von Al, Mg, Zn, Cu, Co, Fe, Ni, Pt, Pd, Gd, Ga oder In ist.

12. Verfahren nach einem der Ansprüche 8-11, dadurch gekennzeichnet, daß die Reaktionstemperatur 120 bis 170°C beträgt.

13. Verfahren nach einem der Ansprüche 8-12, dadurch gekennzeichnet, daß die Umsetzung während 1 Minute bis 12 Stunden erfolgt.

## Claims

1. A process for preparing acid amides, comprising the reaction of an acid with an aliphatic amine in molten urea.

2. The process according to claim 1, characterized in that the acid is a carboxylic, sulfonic or phosphonic acid.

3. The process according to claim 1 or 2, characterized in that the acid is diethylenetriamine pentaacetic acid, diethylenetriamine pentamethylene phosphonic acid, an acid group-containing porphyrine, an acid group-containing phthalocyanine, an acid group-containing naphthalocyanine, an acid group-containing chlorin, an acid group-containing bacteriochlorine, and acid group-containing chlorophyll, an acid group-containing bacteriochlorophyll or a derivative thereof.

4. The process according to any one of claims 1 to 3, characterized in that the amine is a primary or secondary aliphatic amine.

5. The process according to claim 4, characterized in that the amine is tris, glucamine, glucosamine or an aminopolyether.

6. The process according to any one of claims 1 to 5, characterized in that the reaction temperature is 120 to 170°C.

7. The process according to any one of claims 1 to 6, characterized n that the reaction is carried out for 1 minute to 12 hours.

8. The process according to any one of claims 1 to 7, further comprising the reaction of the acid with a metal ion in molten urea.

9. The process for metalating a compound which can be bonded to a metal ion, comprising the reaction of the compound with a metal ion in molten urea, characterized in that the compound is diethylenetriamine pentaacetic acid, diethylenetriamine pentamethylene phosphonic acid, a porphyrine, a phthalocyanine, a naphthalocyanine, a chlorin, a bacteriochlorin, a chlorophyll, a bacteriochlorophyll or a derivative thereof.

10. The process according to claim 9, characterized in that the compound which can bond a metal ion is an acid.

11. The process according to any one of claims 8 to 10, characterized in that the metal ion is an ion of Al, Mg, Zn, Cu, Co, Fe, Ni, Pt, Pd, Gd, Ga or In.

12. The process according to any one of claims 8 to 11, characterized in that the reaction temperature is 120 to 170°C.

13. The process according to any one of claims 8 to 12, characterized in that the reaction is carried out for 1 minute to 12 hours.

## Revendications

1. Procédé destiné à la fabrication d'amides d'acide, englobant la réaction d'un acide avec une amine aliphatique dans une masse d'urée fondue.

2. Procédé selon la revendication 1, caractérisé en ce que l'acide est un acide carboxylique, sulfonique ou phosphorique.

3. Procédé selon la revendication 1 ou 2, caractérisé en ce que l'acide est de l'acide diéthylènetriaminèpenta-acétique, de l'acide diéthylènetriaminepentaméthylènephosphorique, une porphyrine contenant des groupes acides, une phtalocyanine contenant des groupes acides, une naphtalocyanine contenant des groupes acides, une chlorine contenant des groupes acides, une bactériochlorine contenant des groupes acides, une chlorophylle contenant des groupes acides, une bactériochlorophylle contenant des groupes acides ou un dérivé de celles-ci.

4. Procédé selon l'une quelconque des revendications 1 à 3, caractérisé en ce que l'amine est une amine aliphatique primaire ou secondaire.

5. Procédé selon la revendication 4, caractérisé en ce que l'amine est un tris, une glucamine, une glucosamine ou un aminopolyéther.

6. Procédé selon l'une quelconque des revendications 1 à 5, caractérisé en ce que la température de réaction est comprise entre 120 et 170° C.

7. Procédé selon l'une quelconque des revendications 1 à 6, caractérisé en ce que la réaction a lieu pendant 1 minute à 12 heures.

8. Procédé selon l'une quelconque des revendications 1 à 7, englobant en outre la réaction de l'acide avec un ion métallique dans une masse d'urée fondue.

9. Procédé destiné à la métallisation d'un composé pouvant être lié à un ion métallique, englobant la réaction du composé avec un ion métallique dans une masse d'urée fondue, caractérisé en ce que le composé est de l'acide diéthylènetriaminepenta-acétique, de l'acide diéthylènetriamine-pentaméthylène-phosphorique, une porphyrine, une phtalocyanine, une naphtalocyanine, une chlorine, une bactériochlorine, une chlorophylle, une bactériochlorophylle ou un dérivé de celles-ci.

10. Procédé selon la revendication 9, caractérisé en ce que le composé pouvant générer un ion métallique est un acide.

11. Procédé selon l'une quelconque des revendications 8 à 10, caractérisé en ce que l'ion métallique est un ion d'Al, Mg, Zn, Cu, Co, Fe, Ni, Pt, Pd, Gd, Ga ou In.

12. Procédé selon l'une quelconque des revendications 8 à 11, caractérisé en ce que la température de réaction est comprise entre 120 et 170° C.

13. Procédé selon l'une quelconque des revendications 8 à 12, caractérisé en ce que la réaction a lieu pendant 1 minute à 12 heures.
